# EUROPEAN PATENT APPLICATION

(11) **EP 3 545 949 A1**
(43) Date of publication of application: **02.10.2019**
(21) Application number: 18164929.4
(22) Date of filing: 29.03.2018
(51) Int. Cl.: A61K 9/14, A61K 9/48, A61K 31/454

(54) **ORAL DOSAGE FORMS COMPRISING POMALIDOMIDE CRYSTALLINE FORM A**

(71) Applicant: Midas Pharma GmbH, 55218 Ingelheim (DE)
(72) Inventor: Werner, Karl, 64390 Erzhausen (DE); Milak, Spomenka, 8071 Hausmannstätten (AT); Becker, Karin, 82291 Mammendorf (DE); Hussein, Khaled, 81479 München (DE); Dachtler, Markus, 89079 Ulm (DE)
(74) Representative: Drescher, Christian

(57) **Abstract**

The invention relates to oral pharmaceutical compositions comprising Pomalidomide crystalline form A and a stabilizer being selected from sucrose, lactitol, maltitol and mixtures thereof.

## Description

### Field of the invention

Pomalidomide is the international non-proprietary name (INN) of 4- amino-2-(2,6-dioxopiperidine-3-yl)isoindoline-1,3-dione with the following structure:

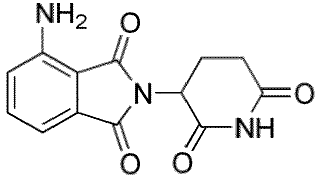

Pomalidomide is currently registered and marketed under the brand name IMNOVID® and POMALYST®, in combination with dexamethasone, for the treatment of multiple myeloma.

A method for the synthesis of Pomalidomide is disclosed in patent application WO 98/03502.

WO 2013/126326 discloses solid forms of Pomalidomide, amongst them crystalline form A. However, this patent application does not provide any data about the characteristics of crystalline form A such as its solubility in standard media. WO 2013/126326 generically discloses long lists of excipients such as disintegrants, fillers, and lubricants in order to formulate Pomalidomide crystalline form A. However, no specific pharmaceutical compositions of crystalline form A are provided, let alone any kind of comparison with the reference market product IMNOVID® or POMALYST®.

The object of the present invention is thus the investigation of suitable pharmaceutical formulations of Pomalidomide crystalline form A. In particular, the object of the present invention is the provision of oral dosage forms of Pomalidomide crystalline form A with improved stability and dissolution characteristics that are similar to IMNOVID® or POMALYST®.

### Summary of the invention

Pomalidomide crystalline form A as characterized in WO 2013/126326 has an X-ray powder diffraction pattern comprising signals at approximately 12, 17 and 26 degrees 2 Theta, in particular signals at approximately 12.1, 14.0, 17.4, 18.4, 24.4 and 25.6 degrees 2 Theta.

It has been found by the inventors of the present invention that some of the excipients that are suggested in WO 2013/126326 for pharmaceutical formulations of Pomalidomide crystalline form A are indeed less favorable than others. For example, Lactose is prone to react with Pomalidomide by a Maillard-reaction. In addition, it has been observed that pharmaceutical formulations of Pomalidomide that contain inorganic phosphates such as dicalcium phosphate, dibasic dicalcium phosphate dihydrate or tribasic dicalcium phosphate in high amounts show degradation of Pomalidomide crystalline form A.

It has also been observed that also microcrystalline cellulose (MCC) in high amounts interacts in some way with Pomalidomide crystalline form A resulting in an incomplete dissolution of the respective pharmaceutical formulations. Since the use of this kind of excipients in pharmaceutical formulations of Pomalidomide crystalline form A requires further R&D efforts, i.e. these excipients are considered less favorable.

On the other hand, pharmaceutical formulations of Pomalidomide crystalline form A that comprise 50% by weight or more of maltitol, sucrose, lactitol or mixtures thereof exhibit a superior stability. It is assumed that maltitol, sucrose, lactitol or mixtures thereof prevent Pomalidomide crystalline form A from degradation and are thus referred to as stabilizers in the following.

Accordingly, in one aspect the current inventions refers to oral pharmaceutical compositions comprising
(a) 0.5% to 5% by weight Pomalidomide crystalline form A,
(b) 50% or more by weight of a stabilizer being selected from maltitol, sucrose, lactitol and mixtures thereof, and
(c) less than 50% by weight of inorganic phosphates and lactose and microcrystalline cellulose,
all percentages by weight being relative to the total weight of the composition.

### Brief description of the drawings

**Figure 1** shows *in-vitro* dissolution tests of pharmaceutical compositions with Pomalidomide crystalline form A according to example 2.
**Figure 2** shows pictures of i) Pomalidomide crystalline form A micronized, ii) maltitol, iii) sucrose and iv) lactitol particles.
**Figure 3** shows *in-vitro* dissolution tests with Pomalidomide form A with different particle size distribution (PSD).
**Figure 4** shows a flow chart of the formulation process for pharmaceutical compositions with Pomalidomide crystalline form A according to example 2.
**Figure 5** shows a comparison of the *in-vitro* dissolution of formulations with microcrystalline cellulose (MCC) according to the reference example with IMNOVID®.

### Detailed description of the invention

To obtain a market authorization for a generic drug development, it is essential to meet several technical and regulatory requirements. The proof of bioequivalence compared to the respective reference product is of major importance. The first step in aiming at bioequivalence is to approach the *in-vitro* dissolution profile of the reference product. In order to do so, it is favorable to establish formulations and processes that allow the adaption of the active substance and excipients as well as of relevant parameters within broad ranges. Therefore, an optimal pharmaceutical formulation process shall be flexible over a broad range of active drug load and also with respect to the excipients and their quantitative amounts.

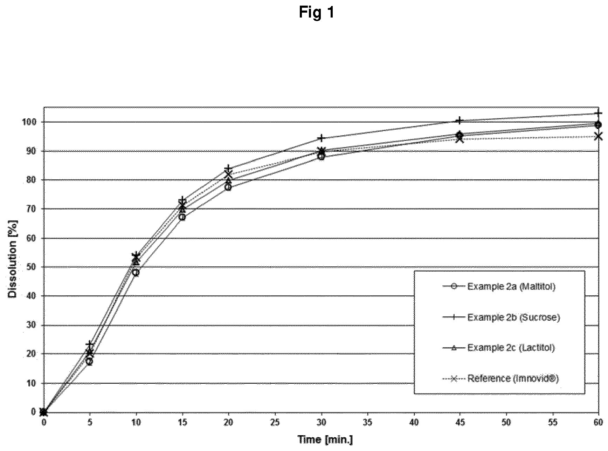

As demonstrated in Fig. 1, the pharmaceutical compositions of the present invention exhibit a quite similar dissolution profile compared to the reference product IMNOVID®.

Accordingly, preferred embodiments of the present invention relate to oral pharmaceutical compositions comprising
(a) from 0.5% to 3% by weight of Pomalidomide crystalline form A,
(b) from 85% to 97% by weight of maltitol, sucrose, lactitol or mixtures thereof,
(c) from 1% to 10% by weight of one or more disintegrant(s),
(d) from 0.1 % to 1.0% by weight of one or more lubricant(s),
(e) optionally other pharmaceutical excipients such as binder(s), glidant(s), filler(s),
(f) less than 15% by weight of inorganic phosphates and lactose and microcrystalline cellulose,
all percentages by weight being relative to the total weight of the capsule.

The terms stabilizer(s) lubricant(s), disintegrant(s), binder(s), glidant(s), filler(s) etc. shall be understood as including a single compound, but also mixtures of compounds.

Pharmaceutically acceptable disintegrant(s) according to the present invention include sodium starch glycolate, crospovidone, croscarmellose sodium and mixtures thereof. Preferred disintegrant is sodium starch glycolate.

Pharmaceutically acceptable lubricant(s) according to the present invention include sodium stearyl fumarate, calcium stearate, stearic acid, stearic acid, glyceryl behenate, hexanedioic acid, hydrogenated vegetable oil, glycerine fumarate and mixtures thereof. Preferred lubricant is sodium stearyl fumarate.

Pharmaceutically acceptable binder(s) according to the present invention include povidone, hydroxypropyl methylcellulose, dihydroxy propyl cellulose, sodium carboxyl methylcellulose, and mixtures thereof.

Pharmaceutical acceptable glidant(s) according to the present invention include colloidal silicone dioxide, talc and magnesium carbonate.

Pharmaceutically acceptable filler(s) according to the present invention include dextrin, kaolin, magnesium oxide, calcium sulfate, citric acid, tartaric acid, fumaric acid, co-polymers from vinyl pyrrolidone and vinyl acetate, co-polymers of polyethylene glycol, and mixtures thereof.

In a particular preferred embodiment of the present invention, the oral pharmaceutical compositions consisting of
(a) from 0.5% to 3% by weight of Pomalidomide crystalline form A,
(b) from 90% to 95% by weight of maltitol, sucrose, lactitol or mixtures thereof,
(c) from 3% to 8% by weight of sodium starch glycolate,
(d) from 0.1% to 0.5% by weight of sodium stearyl fumarate,

all percentages by weight being relative to the total weight of the composition.

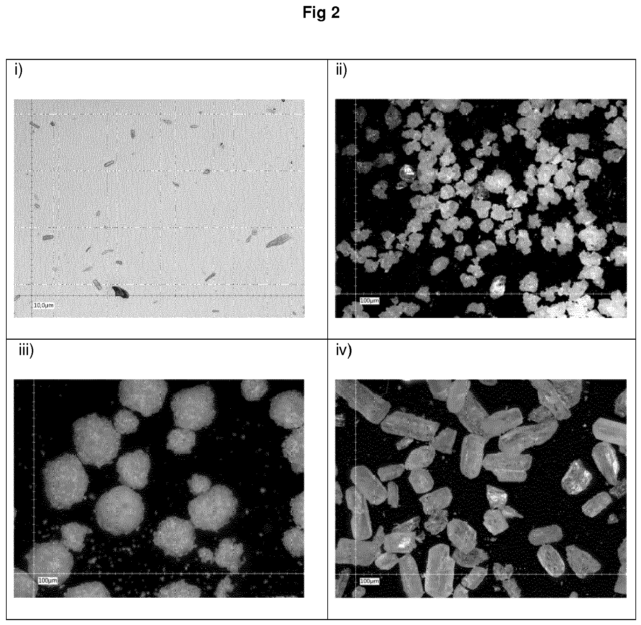

It has also been found by the inventors of the present invention that favorable pharmaceutical compositions comprise micronized Pomalidomide crystalline form A with a particle size of D₅₀ below 15µm, whereas the stabilizer(s) should not exhibit a particle size with a D₅₀ the exhibit a D₅₀ of above 500µm (Fig. 2).

Preferably, the Pomalidomide crystalline form A exhibits a particle size with a D₅₀ ranging from 3µm to 10µm, even more preferably the D₅₀ of ranges from 5µm to 8µm. The stabilizer(s) preferably exhibit a particle size with a D₅₀ ranging from 150µm to 450µm, even more preferably the D₅₀ of ranges from 200µm to 400µm.

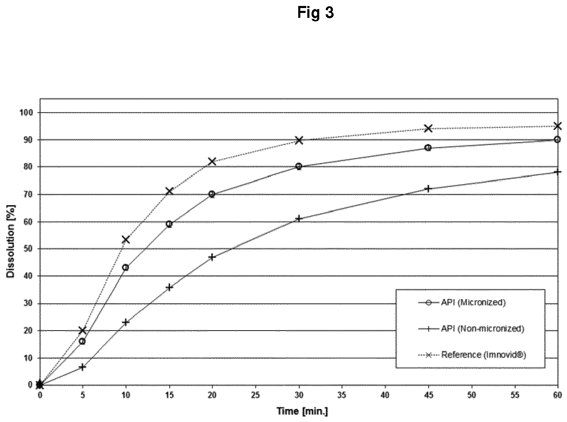

As demonstrated in Fig. 3, the dissolution profile of micronized Pomalidomide crystalline form A does match the dissolution profile reference product Imnovid® better than unmicronized.

It has further been observed that the processability of mixtures of Pomalidomide crystalline form A and sucrose, lactitol and/or maltitol is better if the PSD (D₅₀) of the excipients compared to the PSD (D₅₀) of Pomalidomide crystalline form A remains within a certain ratio. The ratio of the PSD of the stabilizer compared to the PSD of Pomalidomide crystalline form A ranges from 100:1 to 10:1, preferably from 80:1 to 15:1, most preferred from 60:1 to 30:1.

Accordingly, a particular preferred embodiment of the present invention relates to oral pharmaceutical compositions consisting of
(a) from 0.5% to 3% by weight of Pomalidomide crystalline form A with a D₅₀ ranging from 3µm to 10µm, preferably ranging from 5µm to 8µm,
(b) from 90% to 95% by weight of maltitol, sucrose, lactitol or mixtures thereof with a D₅₀ ranging from 150µm to 450µm, preferably ranging from 200µm to 400µm,
(c) from 1% to 10% by weight of one or more disintegrant(s),
(d) from 0.1 % to 1.0% by weight of one or more lubricant(s),
all percentages by weight being relative to the total weight of the composition, wherein the ratio of the PSD of the stabilizer compared to the PSD of Pomalidomide crystalline form A ranges from 100:1 to 10:1, preferably from 80:1 to 15:1, most preferred from 60:1 to 30:1.

Preferably the oral pharmaceutical compositions with Pomalidomide crystalline form A according to the present invention are in form of capsules.

Preferably the oral pharmaceutical compositions comprise 1 mg, 2mg, 3mg or 4mg of Pomalidomide crystalline form A.

The oral pharmaceutical compositions with Pomalidomide crystalline form A according to the present invention may be prepared by formulation processes well known in the art.

### Examples

### Example 1: Binary mixtures

Binary mixtures of 50% by weight of Pomalidomide crystalline form A and 50% by weight of excipient are prepared my simple mixing. The composition of the binary mixtures is displayed table 1:

**Table 1**

| | **Example 1a** | **Example 1b** | **Example 1c** | **Example 1d** | **Example 1e** | **Example 1f** |
|---|---|---|---|---|---|---|
| **Pomalidomide crystalline form A** | 3.5 mg | 3.5 mg | 3.5 mg | 3.5 mg | 3.5 mg | 3.5 mg |
| **Maltitol** | 3.5 mg | - | - | - | - | - |
| **Sucrose** | - | 3.5 mg | - | - | - | - |
| **Lactitol** | - | - | 3.5 mg | - | - | - |
| **CaHPO₄** | - | - | - | 3.5 mg | - | - |
| **CaHPO_{4*}2H₂O** | - | - | - | - | 3.5 mg | - |
| **Ca₃(PO₄)₂** | - | - | - | - | - | 3.5 mg |

The binary mixtures of examples 1 a-f were stored for up to 12 weeks under accelerated temperature and relative humidity conditions (50 ºC / 80% RH) and then analyzed. Conditions for assay and purity determination by HPLC are displayed in table 2.

**Table 2**

| | |
|---|---|
| **Analytical column:** | Phenomenex Synergi 4u Polar-RP 4.6x150mm |
| **Mobile phase A:** | 0.1% TFA |
| **Mobile phase B:** | Acetonitrile |
| **Flow rate:** | 1.0 ml/min |
| **Column temperature:** | 20°C |
| **Detection wavelength:** | 240 nm |
| **Injection volume** | 10 µl |
| **Gradient**: | |
| **Time** | |
| **0** | 80% B / 20% A |
| **5 min** | 80% B / 20% A |
| **35 min** | 20% B |
| **35.1 min** | 80% B |

The total impurities (in % wt/wt) of the binary mixtures according to example 1 are displayed in table 3.

**Table 3**

| **Binary mixture** | **Initial** | **2 weeks** | **4 weeks** | **12 weeks** |
|---|---|---|---|---|
| Ex. 1a (Maltitol) | < RL | < RL | < RL | < RL |
| Ex. 1b (Sucrose) | < RL | < RL | < RL | < RL |
| Ex. 1c (Lactitol) | < RL | < RL | < RL | < RL |
| Ex. 1d (CaHPO₄ | < RL | 0.05% wt/wt | 0.11% | not further analyzed |
| Ex. 1e (CaHPO_{4*}2H₂O) | < RL | < RL | 0.06% | not further analyzed |
| Ex. 1f (Ca₃(PO₄)₂₎ | < RL | 0.32% | 0.60% | not further analyzed |

As can be concluded from table 3, binary mixtures of Pomalidomide crystalline form A with inorganic phosphates show an increase of degradation products during the stability tests, whereas mixtures of Pomalidomide crystalline form A with maltitol, sucrose and lactitol exhibit an excellent stability.

### Example 2: Pharmaceutical formulations

The composition of the formulations according to the present invention comprising Pomalidomide in crystalline form A is displayed table 4:

**Table 4**

| **Excipient** | **Function** | **Example 2a** | | **Example 2b** | | **Example 2c** | |
|---|---|---|---|---|---|---|---|
| | | **Quantity** | | **Quantity** | | **Quantity** | |
| Pomalidomide form A | Drug Substance | 3mg | 1.0% | 3mg | 1.1% | 3mg | 0.8% |
| Maltitol | Stabilizer | 274.8 mg | 93.8% | - | - | - | - |
| Sucrose | | - | - | 250.2 mg | 93.7% | - | - |
| Lactitol | | - | - | - | - | 355.4 mg | 94.0% |
| Sodium starch glycolate | Disintegrant | 14.6 mg | 5.0% | 13.4 mg | 5.0% | 19.0 mg | 5.0% |
| Sodium stearyl fumarate | Lubricant | 0.6 mg | 0.2% | 0.6 mg | 0.2% | 0.8 mg | 0.2% |
| TOTAL | | 293 mg | 100.0% | 267.2 mg | 100.0% | 378.2 mg | 100.0% |

The process for the manufacture of formulations of example 2 comprises the following steps:
(a) The drug substance was sieved
(b) The stabilizer(s) and disintegrant(s) are mixed and sieved
(c) A portion (10%) of stabilizer(s) and disintegrant(s) are added to the API
(d) The mixture was mixed in a turbula mixer
(e) The remaining portion of stabilizer(s) are added and the resulting reaction mixture was mixed in a turbula mixer
(f) The lubricant(s) are sieved and added to the reaction mixture
(g) The reaction mixture was mixed in a turbula mixer
(h) The final blend was filled into capsules.

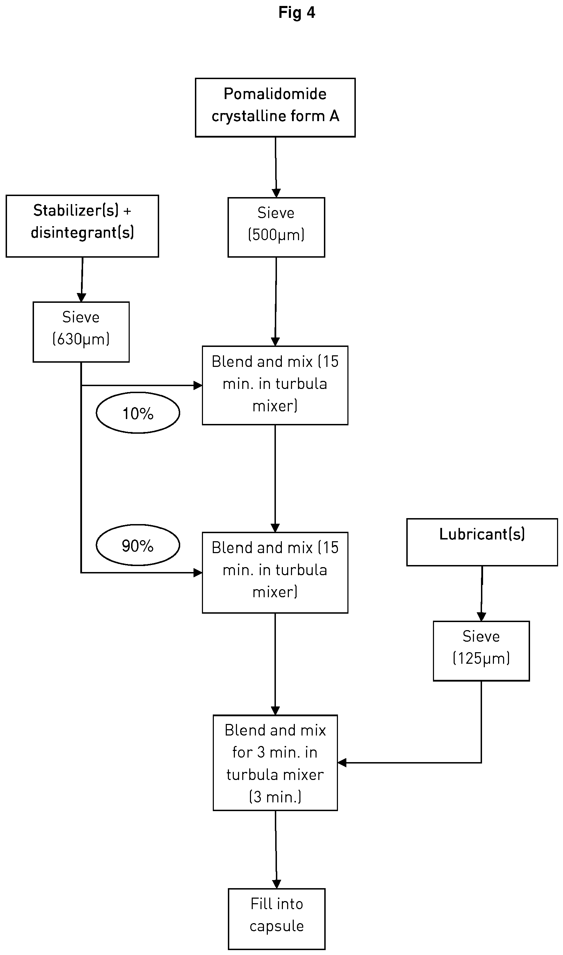

A flow chart with detailed process parameters is displayed in Fig. 4.

The particle size distribution of Pomalidomide crystalline form A and the stabilizer(s) are measured by laser diffraction analysis, using the equipment and procedure as displayed in table 5:

**Table 5**

| | |
|---|---|
| **Apparatus** | Mastersizer 2000, Hydro 2000S wet dispersion unit, Malvern |
| **Technique used** | Dispersion method |
| **Parameters** | - Stirrer speed: 2500 rpm |
| | - Sonication: no internal sonication |
| | - Background time: 5 sec |
| | - Measurement time: 10 sec |
| | - Measurement cycles: 3 |
| | - Red light only: no |
| | - Particle refractive index: 1.520 |
| | - Dispergant refractive index: 1.403 |
| | - Absorption: 1.0 |
| **Dispersion medium** | Silicone oil |
| **Sample preparation** | About 30 mg of the API are transferred into about 30 ml of silicone oil. Sonicate for about 30 sec in an external ultrasonic bath while stirring slightly. The sample is added to the dispersant in the measurement cell until at least the lower obscuration limit is met. |

The particle size distribution (PSD) of Pomalidomide crystalline form A and the stabilizer(s) according to the present invention are displayed in table 6:

**Table 6**

| **PSD** | **API (non-micronized)** | **API (micronized)** | **Maltitol** | **Sucrose** | **Lactitol** |
|---|---|---|---|---|---|
| **D₁₀** | 5.4 µm | 1.7 µm | 123 µm | 185 µm | 170 µm |
| **D₅₀** | 18.0 µm | 6.9 µm | 213µm | 365 µm | 313 µm |
| **D₉₀** | 49.4 µm | 14.5 µm | 342 µm | 625 µm | 535 µm |

The *in-vitro* dissolution of tablets (30mg) according to example 2 were analyzed according to USP app. II- Method 2 (paddle, spirale sinker) with the settings as displayed in table 7.

**Table 7**

| | |
|---|---|
| **Medium** | HCl 0.1N |
| **Volume** | 900ml |
| **Temperature** | 37.0ºC ± 0.5ºC |
| **Stirring speed** | 50 rpm |

The dissolution profiles of the formulations according to example 2 are displayed in table 8 and Fig. 1.

**Table 8**

| **Time** (min) | **Ex. 2a** | **SD** | **Ex. 2b** | **SD** | **Ex. 2c** | **SD** | **Imnovid®** | **SD** |
|---|---|---|---|---|---|---|---|---|
| 0 | 0 | 0 | 0 | 0 | 0 | 0 | 0 | 0 |
| 5 | 17.3 | 3.9 | 23.2 | 1.3 | 21.1 | 2.2 | 20.2 | 0.9 |
| 10 | 48.0 | 6.1 | 54.1 | 1.5 | 51.8 | 1.0 | 53.2 | 1.9 |
| 15 | 67.1 | 6.6 | 73.1 | 1.4 | 69.9 | 0.8 | 71.3 | 2.2 |
| 20 | 77.4 | 5.9 | 83.9 | 2.8 | 79.8 | 1.3 | 81.8 | 2.6 |
| 30 | 87.9 | 3.9 | 94.3 | 4.1 | 90.1 | 1.7 | 89.8 | 2.7 |
| 45 | 95.2 | 3.4 | 100.6 | 4.6 | 95.9 | 2.1 | 94.1 | 2.9 |
| 60 | 98.8 | 3.6 | 103.1 | 4.8 | 99.5 | 2.9 | 95.0 | 2.9 |

All tested compositions release at least about 85% of the Pomalidomide crystalline form A from the composition in no later than 30 minutes from the start of the test. At least 95% of the Pomalidomide crystalline form A are dissolved in no later than 60 minutes from the start of the test. A comparison with the reference product IMNOVID® showed a practically identical dissolution profile.

### Comparative example

Pharmaceutical compositions were prepared as follows:
(a) Pomalidomide crystalline form A and isomalt were mixed and sieved through 250 µm mesh size
(b) Half of the microcrystalline cellulose was combined with the croscarmellose sodium and the fumed silica and added to the pre-mix of step (a)
(c) After blending, the mixture was sieved through 250 µm mesh size.
(d) The remaining part of MCC and the sodium stearyl fumarate were added and the mixture was blended.
(e) The final blend of step (d) was filled into capsules.

The composition of the resulting formulation is displayed table 9:

**Table 9**

| **Excipient** | **Function** | **Quantity** | |
|---|---|---|---|
| Pomalidomide form A | Drug Substance | 3 mg | 1.25 % |
| Microcrystalline cellulose (MCC) | Filler | 195.0 mg | 81.25 % |
| Isomalt | Stabilizer | 30.0 mg | 12.5 % |
| Fumed silica | Glidant | 1.2 mg | 0.5 % |
| Croscarmellose sodium | Disintegrant | 7.2 mg | 3.0 % |
| Sodium stearyl fumarate | Lubricant | 3.6 mg | 1.5 % |
| TOTAL | | 293 mg | 100.0 % |

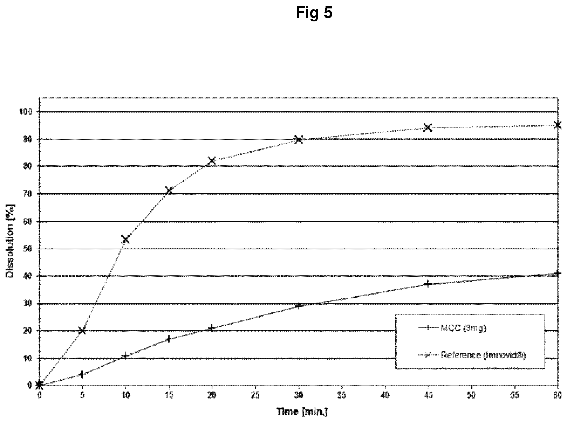

As displayed in fig. 5, the dissolution of pharmaceutical composition according to the comparative example is incomplete which is due to the presence of MCC.

## Claims

1. Oral pharmaceutical composition comprising
(a) from 0.5 % to 5 % by weight Pomalidomide crystalline form A,
(b) 50 % or more by weight of a stabilizer being selected from maltitol, sucrose, lactitol and mixtures thereof,
(c) less than 50 % by weight of inorganic phosphates and lactose and microcrystalline cellulose,
all percentages by weight being relative to the total weight of the composition.

2. Oral pharmaceutical composition according claim 1 comprising
(b) 70 % or more by weight of the stabilizer, and
(c) 30 % or less by weight of inorganic phosphates and lactose and microcrystalline cellulose.

3. Oral pharmaceutical composition according claim 1 or claim 2 comprising
(b) 85 % or more by weight of the stabilizer, and
(c) 15 % or less by weight of inorganic phosphates and lactose and microcrystalline cellulose.

4. Oral pharmaceutical composition comprising
(a) from 0.5 % to 3 % by weight of Pomalidomide crystalline form A,
(b) from 85 % to 97 % by weight of maltitol, sucrose, lactitol or mixtures thereof,
(c) from 1 % to 10 % by weight of one or more disintegrant(s),
(d) from 0.1 % to 1.0 % by weight of one or more lubricant(s),
(e) optionally other pharmaceutical excipients such as binder(s), glidant(s), filler(s),
(f) less than 15% by weight of inorganic phosphates and lactose and microcrystalline cellulose,
all percentages by weight being relative to the total weight of the composition.

5. Oral pharmaceutical composition according to claim 4 wherein the one or more disintegrant(s) is selected from the group of sodium starch glycolate, crospovidone, croscarmellose sodium, and mixtures thereof.

6. Oral pharmaceutical composition according to claim 4 or claim 5 wherein the one or more lubricant(s) is selected from the group of sodium stearyl fumarate, calcium stearate, stearic acid, stearic acid, glyceryl behenate, hexanedioic acid, hydrogenated vegetable oil and glycerine fumarate, and mixtures thereof.

7. Oral pharmaceutical composition consisting of
(a) from 0.5% to 3% by weight of Pomalidomide crystalline form A,
(b) from 90% to 95% by weight of maltitol, sucrose, lactitol or mixtures thereof,
(c) from 3% to 8% by weight of sodium starch glycolate,
(d) from 0.1% to 0.5% by weight of sodium stearyl fumarate,
all percentages by weight being relative to the total weight of the composition.

8. Oral pharmaceutical composition according to any of the preceding claims, wherein the Pomalidomide crystalline form A exhibits a D₅₀ ranging from 3µm to 10µm, preferably ranging from 5µm to 8µm.

9. Oral pharmaceutical composition according to any of the preceding claims, wherein the stabilizer(s) exhibit a particle size with a D₅₀ ranging from 150µm to 450µm, preferably ranging from 200µm to 400µm.

10. Oral pharmaceutical composition according to any of the preceding claims, wherein the ratio of the PSD of the stabilizer compared to the PSD of Pomalidomide crystalline form A ranges from 100:1 to 10:1, preferably from 80:1 to 15:1, most preferred from 60:1 to 30:1.

11. Oral pharmaceutical composition consisting of
(a) from 0.5% to 3% by weight of Pomalidomide crystalline form A with a D₅₀ ranging from 3µm to 10µm, preferably ranging from 5µm to 8µm,
(b) from 90% to 95% by weight of one or more stabilizer(s) being selected from maltitol, sucrose, lactitol with a D₅₀ ranging from 150µm to 450µm, preferably ranging from 200µm to 400µm,
(c) from 1% to 10% by weight of one or more disintegrant(s),
(d) from 0.1 % to 1.0% by weight of one or more lubricant(s),
all percentages by weight being relative to the total weight of the composition, **characterized in that** the ratio of the PSD of the stabilizer(s) compared to the PSD of Pomalidomide crystalline form A ranges from 100:1 to 10:1, preferably from 80:1 to 15:1, most preferred from 60:1 to 30:1.

12. Oral pharmaceutical composition according to any of the preceding claims, wherein Pomalidomide crystalline form A is **characterized by** having an X-ray powder diffraction pattern comprising signals at 12, 17 and 26 ±0.5 degrees 2 Theta.

13. Oral pharmaceutical composition according to any of the preceding claims, wherein Pomalidomide crystalline form A is **characterized by** having an X-ray powder diffraction pattern comprising signals at 12.1, 14.0, 17.4, 18.4, 24.4 and 25.6 ±0.2 degrees 2 Theta.

14. Oral pharmaceutical composition according to any of the preceding claims in form of a capsule.

15. Oral pharmaceutical composition according to any of the preceding claims, comprising 1 mg, 2mg, 3mg or 4mg of Pomalidomide crystalline form A.
